# EUROPEAN PATENT SPECIFICATION

(11) **EP 1 192 969 B1**
(45) Date of publication and mention of the grant of the patent: **16.02.2005**
(21) Application number: 01117251.7
(22) Date of filing: 17.07.2001
(51) Int. Cl.: A61M 21/00, A47K 3/28, A61H 23/02, A61H 35/00, A61H 33/00, A47K 3/40

(54) **Fitted shower booth with loudspeaker**
Duschkabine mit Lautsprecher
Cabine de douche avec haut-parleur

(30) Priority: 29.09.2000 IT PN000058
(43) Date of publication of application: 03.04.2002
(73) Proprietor: DOMINO S.r.L., 33097 Spilimbergo, Pordenone (IT)
(72) Inventor: Colussi, Lucio, 33072 Casarsa, Pordenone (IT); Cadamuro, Antonio, 33097 Spilimbergo, Pordenone (IT)
(74) Representative: Giugni, Valter

(56) References cited:
- WO-A-98/27923
- DE-A- 3 207 892
- DE-C- 19 911 376
- GB-A- 2 337 697
- US-A- 5 388 162

## Description

The present invention refers to a shower booth of the fitted type, ie. of the type that is variously equipped in view of inducing in the user definite psycho-physical sensations of well-being through the possibly combined delivery of water, vapours, scents, sounds, etc.

As this is known for instance from the disclosure in EP-A-0 986 985, it actually is relatively easy for a bath-tub to be equipped with various devices adapted to bring about a synchronized combination of external actions capable of exerting the above mentioned sensations of well-being on the entire human body. Such external actions may in particular be constituted by the delivery of water, scents, musical sounds, light or chromatic rays and, above all, jets of an air/water mixture capable of performing an important massaging action on the body.

WO 98/27923 discloses an entertainment and treatment system comprising a bathtub having a wall forming a water reservoir, a transducer mounted on an exterior surface of the wall for transmitting energy to the wall, and an excitation source for energizing the transducer; the bathtub forms an integral part of a loudspeaker in which the wall transmits vibrations responsive to operation of the transducer, the vibrations being emitted from an interior surface of the wall into the water reservoir.

DE 32 07 892 discloses a footbath tub having one or more ultrasound-generating heads, which are firmly or detachably mounted on the tub sounding board and/or the side walls; they radiate ultrasound into the bath water and onto the treated feet such that it penetrates deeply into the tissue and combats disorders of the muscles, joints, ligaments and skin of the feet in connection with an air bubble system which may be built in.

In a shower booth, on the contrary, owing to the geometry and the reduced internal dimensions thereof, as well as the fact that the user is not immersed in a mass of water, it proves quite difficult to effectively transmit to the entire body of the same user all of the above cited external actions, and much more so in a combined manner. In particular, it proves difficult to effectively use the traditional water delivery spouts in view of obtaining such a massaging action on the feet, which are a notoriously important part of the human body in view of a beneficial relaxing action.

It would therefore be desirable, and it is actually a main purpose of the present invention, to provide a fitted-type shower booth that is equipped with simple and reliable means adapted to perform an effective plantar massage action, in such a manner as to substantially compensate for the above cited reduced possibilities of transmitting to the user external actions adapted to induce psycho-physical sensations of well-being.

In particular, it is a purpose of the present invention to provide a fitted shower booth of the above cited kind, with which said plantar massage is capable of being performed in a correct combination, even in synchronism, with other external actions such as the delivery of water, musical sounds, scents, light rays, and the like.

A further purpose of the present invention is to provide a fitted shower booth of the above cited kind, in which the plantar massage is capable of being performed at rapidly variable vibration frequencies comprised within a very wide spectrum.

According to the present invention, these aims are reached in a fitted-type shower booth as defined in claim 1 and further embodiments as recited in the appended claims.

The features and advantages of the present invention will anyway be more readily and clearly understood from the description that is given below by way of non-limiting example with reference to the accompanying drawings, in which:

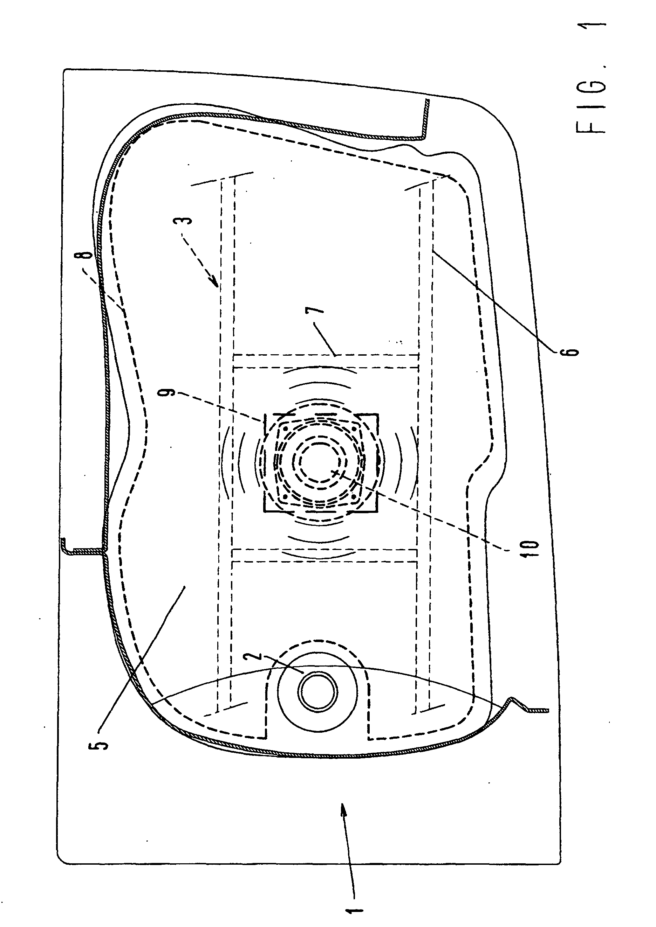

- Figure 1 is a schematical top view, as sectioned in correspondence of the base, of a preferred embodiment of the shower booth according to the present invention; and

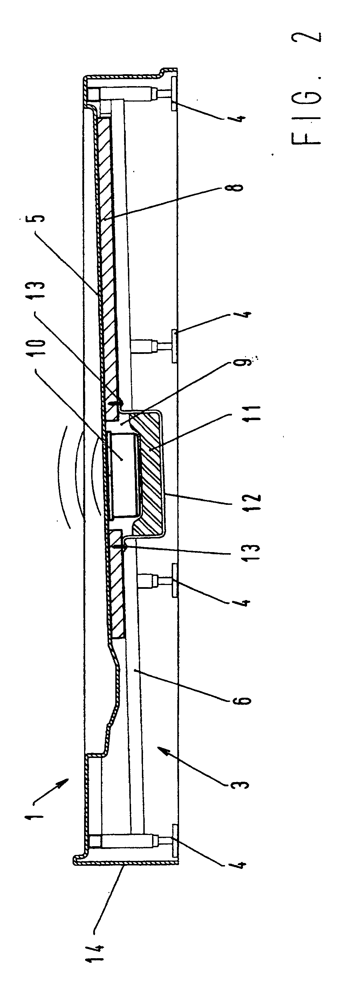

- Figure 2 is a schematical, cross-sectional view of the base of the shower booth illustrated in Figure 1.

With reference to the above cited Figures, the shower booth mainly comprises a base pan 1 that is substantially arranged flush with the walls (not shown for reasons of greater descriptive simplicity) defining the side surface of the same booth.

In particular, the base pan 1 can be made of ABS, methacrylate ester or any similar material, and may be variously shaped according to the particular needs, with a thin top surface 5 that is inclined, ie. sloping towards a drain 2 and smoothly links up with a side surface 14 extending down to the floor.

The base pan 1 is supported by a rigid frame 3 adapted to rest on the floor through adjustable resting feet 4 and comprising cross-members 6, 7 on which said top surface 5 of the pan is capable of resting through the interposition of at least a panel 8.

This panel comprises at least an aperture 9, in correspondence of which there is provided a loudspeaker 10 arranged below said top surface 5 and oriented with its front face (ie. the sound emitting face) towards the same top surface.

In a preferred manner, the loudspeaker 10 is placed, in elastic contact therewith, between the top surface 5 and a body 11 of sound deadening material, such as for instance closed-cell cellular plastics, or the like, which is in turn mounted in a cup-like container 12 provided therebelow. The upper rim of such a container is flanged and removably attached to said panel 8 by means of screws 13 or similar fastening means capable of being loosened in view of allowing for possibly required maintenance operations to be carried out on the component parts involved.

In short, said top surface 5 of the base pan 1 forms, jointly with the panel 8, a substantially rigid footboard adapted to be caused to vibrate, in such a manner as to perform an effective plantar massage, by the acoustic waves generated by the loudspeaker 10.

In this connection, the panel 8 is preferably made of a sufficiently rigid material such as wood, and may be fixed to both the top surface 5 and the support cross-members 6, 7 by elastic bonding thereto. The substantial rigidity of the panel 8 limits the absorption and/or the damping of the acoustic waves, which can therefore be effectively used to cause the footboard 5, 8 to vibrate, not only in correspondence of the aperture 9, but also of the whole panel 8.

The effectiveness of the phono-vibratory system of the footboard 5, 8 is further enhanced by an optimum directionality , since the container 12 enclosing the loudspeaker 10 acts as a resonance box, and the sound-deadening material 11, further to supporting the loudspeaker 10 mechanically, prevents the sound from undesirably scattering in other directions differing from the desired one, ie. the direction towards the footboard 5, 8.

The loudspeaker 10 shall of course be understood as being connected to a per sè known (and therefore not shown) acoustical driving system, thanks to which the loudspeaker itself is able to diffuse musical sounds inside the booth and, at the same time, cause the footboard 5, 8 to vibrate so as to perform a corresponding plantar massage.

It should be noticed that, other than in traditional electromechanical vibrating systems, which are largely known as being complex, expensive and, above all, limited by the movement of mechanical members, the acoustically vibrating system according to the present invention is substantially free from limitations and constraints connected to the vibratory frequencies of the footboard 5, 8, which can also be caused to vary in an extremely rapid manner. This practically enables not only plantar massages to be performed that can be varied according to the requirements, for instance in tune with musical melodies, but also the vibrations of the footboard to be freely synchronized in an optimum manner with the frequencies of other treatment agents (eg. the colours of light rays) which the fitted shower booth is capable of delivering in an inherently known manner.

It will be appreciated that the afore described shower booth may be subject to a number of modifications and variants without departing from the scope of the present invention.

## Claims

1. Shower booth comprising a base pan (1) having a top surface (5) comprising at least a substantially rigid panel (8) provided below said top surface (5), said panel (8) comprising at least an aperture (9), in correspondence of which a loudspeaker (10) is arranged below said top surface (5) with its front face facing said top surface (5), at least a portion of said top surface (5) forming a substantially rigid footboard adapted to be caused to vibrate, so as to perform a plantar massage, by the acoustic waves generated by said loudspeaker (10) coupled to said footboard, **characterized in that** said panel (8) is fixed by elastic bonding both to said top surface (5) and to support members (6, 7) provided below said panel (8) as a support to said top surface (5).

2. Shower booth according to claim 1, **characterized in that** said loudspeaker (10) is housed in a cup-shaped container (12) having its rim attached to said panel (8) in correspondence of said aperture (9).

3. Shower booth according to claim 2, **characterized in that** said loudspeaker (10) is arranged between said top surface (5) and a body (11) of sound-deadening material accommodated in said container (12) so as to minimize acoustic scattering.

4. Shower booth according to claim 3, **characterized in that** said loudspeaker (10) is arranged between said top surface (5) and said body of sound-deadening material (11) in an elastic contact therewith.

5. Shower booth according to claim 1, **characterized in that** said panel (8) is made of wood.

6. Shower booth according to claim 1, **characterized in that** said panel (8) is interposed between said top surface (5) and said support members (6, 7) on which said top surface (5) is capable of resting.

## Patentansprüche

1. Duschkabine, die eine Bodenwanne (1) mit einer Oberseite (5) umfasst, die wenigstens eine im Wesentlichen starre Platte (8) umfasst, die unter der oberen Fläche (5) vorhanden ist, wobei die Platte (5) wenigstens eine Öffnung (9) umfasst, an der ein Lautsprecher (10) unter der oberen Fläche (5) angeordnet ist, wobei seine Vorderseite der oberen Fläche (5) zugewandt ist und wenigstens ein Abschnitt der oberen Fläche ein im Wesentlichen starres Trittbrett bildet, das so eingerichtet ist, dass es durch die Schallwellen, die von dem Lautsprecher (10) erzeugt werden, der mit dem Trittbrett verbunden ist, in Schwingung versetzt wird, um eine Fußsohlenmassage durchzuführen, **dadurch gekennzeichnet, dass** die Platte (8) durch elastische Bindung sowohl an der oberen Fläche (5) als auch an Trageelementen (6, 7) befestigt ist, die unter der Platte (8) vorhanden sind, um die obere Fläche (5) zu tragen.

2. Duschkabine nach Anspruch 1, **dadurch gekennzeichnet, dass** der Lautsprecher (10) in einem schalenförmigen Behälter (12) aufgenommen ist, dessen Rand an der Öffnung (9) an der Platte (8) angebracht ist.

3. Duschkabine nach Anspruch 2, **dadurch gekennzeichnet, dass** der Lautsprecher (10) zwischen der oberen Fläche (5) und einem Körper (11) aus schalldämpfendem Material angeordnet ist, das in der Kammer (12) aufgenommen ist, um Schallstreuung auf ein Minimum zu verringern.

4. Duschkabine nach Anspruch 3, **dadurch gekennzeichnet, dass** der Lautsprecher (10) zwischen der oberen Fläche (5) und dem Körper (11) aus schalldämpfendem Material (11) in elastischem Kontakt damit angeordnet ist.

5. Duschkabine nach Anspruch 1, **dadurch gekennzeichnet, dass** die Platte (8) aus Holz besteht.

6. Duschkabine nach Anspruch 1, **dadurch gekennzeichnet, dass** die Platte (8) zwischen der oberen Fläche (5) und den Trageelementen (6, 7) angeordnet ist, auf denen die obere Fläche (5) aufliegen kann.

## Revendications

1. Cabine de douche comprenant un bac de base (1) ayant une surface supérieure (5) comprenant au moins un panneau sensiblement rigide (8) prévu au-dessous de ladite surface supérieure (5), ledit panneau (8) comprenant au moins une ouverture (9) en correspondance de laquelle un haut-parleur (10) est placé au-dessous de ladite surface supérieure (5) avec sa face frontale tournée vers ladite surface supérieure (5), au moins une partie de ladite surface supérieure (5) formant un appuie-pieds sensiblement rigide adapté pour être mis en vibrations, afin d'appliquer un massage plantaire, par les ondes acoustiques générées par ledit haut-parleur (10) couplé audit appuie-pieds, **caractérisée en ce que** ledit panneau (8) est fixé par liaison élastique à la fois à ladite surface supérieure (5) et à des éléments supports (6, 7) prévus au-dessous dudit panneau (8) en tant que supports de ladite surface supérieure (5).

2. Cabine de douche selon la revendication 1, **caractérisée en ce que** ledit haut-parleur (10) est logé dans un récipient (12) en forme de coupe ayant son bord fixé audit panneau (8) en correspondance de ladite ouverture (9).

3. Cabine de douche selon la revendication 2, **caractérisée en ce que** ledit haut-parleur (10) est placé entre ladite surface supérieure (5) et un corps (11) en matériau d'amortissement phonique placé dans ledit récipient (12) afin de minimiser la diffusion acoustique.

4. Cabine de douche selon la revendication 3, **caractérisée en ce que** ledit haut-parleur (10) est placé entre ladite surface supérieure (5) et ledit corps en matériau d'amortissement acoustique (11) en contact élastique avec celui-ci.

5. Cabine de douche selon la revendication 1, **caractérisée en ce que** ledit panneau (8) est fabriqué en bois.

6. Cabine de douche selon la revendication 1, **caractérisée en ce que** ledit panneau (8) est intercalé entre ladite surface supérieure (5) et lesdits éléments supports (6, 7) sur lesquels ladite surface supérieure (5) peut reposer.
